# EUROPÄISCHE PATENTSCHRIFT

(11) **EP 2 837 618 B1**
(45) Veröffentlichungstag und Bekanntmachung des Hinweises auf die Patenterteilung: **06.03.2019**
(21) Anmeldenummer: 14177090.9
(22) Anmeldetag: 15.07.2014
(51) Int. Cl.: C07C 45/74, C07C 45/82

(54) **Hydrolyse der bei der Produktion von Isophoron anfallenden Rückstände zur Rückgewinnung von Isophoron und Aceton**
Hydrolysis of the residues of production of isophorone for the recovery of isophorone and acetone
Hydrolyse de résidus issus de la production d'isophorone pour la production d'isophorone et acétone

(30) Priorität: 12.08.2013 DE 102013215874
(43) Veröffentlichungstag der Anmeldung: 18.02.2015
(73) Patentinhaber: Evonik Degussa GmbH, 45128 Essen (DE)
(72) Erfinder: Nitz, Jörg-Joachim, 45257 Essen (DE); Kohlstruk, Stephan, Dr., 48249 Dülmen (DE); Jansen, Robert, 46244 Bottrop (DE); Orschel, Matthias, Dr., 48161 Münster (DE); Merkel, Andreas, 45665 Recklinghausen (DE); Demming, Michael, 48249 Dülmen (DE); Mendorf, Matthias, Dr., 44329 Dortmund (DE); Döring, Jens, Dr., 44141 Dortmund (DE); Hengstermann, Axel, Dr., 48308 Senden (DE); Hoff, Andreas, Dr., 59889 Eslohe (DE); Müller, Anja, Dr., 44135 Dortmund (DE)
(74) Vertreter: Evonik Patent Association

(56) Entgegenhaltungen:
- DE-A1- 2 520 681
- DE-A1- 2 645 281
- DE-A1-102010 062 587
- DE-A1-102011 075 777

## Beschreibung

Die Erfindung betrifft ein Verfahren zur Herstellung von Isophoron (3,5,5-Trimethyl-2-cyclohexen-1-on).

Die Erfindung betrifft insbesondere eine Erweiterung des Verfahrens zur Herstellung von Isophoron zur verbesserten Rohstoffnutzung. Dabei werden die anfallenden organischen Rückstände erneut hydrolysiert (Hydrolyse 2), um die sich dabei bildenden Wertstoffe in den Prozess zurückzuführen. Unter Wertstoff versteht man neben Aceton und Isophoron, insbesondere auch die Zwischenprodukte Phoron, Mesityloxid und Diacetonalkohol.

Isophoron wird u. a. als hochsiedendes Lösemittel in der Lack-, Druckfarben-, Klebstoff- und der Pflanzenschutzmittel-Industrie verwendet. Nach dem bekannten Stand der Technik kann das Isophoron weiter verarbeitet werden bspw. zu Isophoronnitril, Isophorondiamin, Isophorondiisocyanat oder Keto-Isophoron.

Bei Isophoron handelt es sich um das trimere Kondensationsprodukt des Acetons. Die Herstellung von Isophoron erfolgt über eine katalysierte Aldol-Kondensation von Aceton.

Die Reaktion in der Flüssigphase wird in der Patentliteratur nahezu ausschließlich unter alkalischen Bedingungen bei erhöhten Temperaturen und hohen Drücken beschrieben.

In den Patentdokumenten der Hibernia Chemie (DE 10 95 818, DE 11 44 269, DE 12 05 525, DE 11 65 018) aus den 1960er Jahren wird neben dem Einsatz eines einphasigen Edukt / Katalysator-Gemisches mit niedrigen Lauge-Konzentrationen auch die Aufarbeitung mittels Hydrolysekolonne beschrieben. Isophoron wird hier in einem Druckreaktor durch Kondensation von Aceton in flüssiger Phase mittels Alkalimengen (NaOH oder KOH) von weniger als 1 % als Katalysator und unter Anwendung von Wassermengen von unterhalb 20 % bei Temperaturen von 150 - 250 °C hergestellt. Die sich bei der Reaktion ausbildenden zwei Phasen werden sowohl durch eine geeignete Reaktionsführung (Reaktorbau, Impulsgenerator), als auch durch den Einsatz eines Emulgators emulgiert, um einen guten Kontakt zwischen Katalysator und den Reaktanten zu gewährleisten (DE 10 95 818).

Daneben wird in DE 12 05 525 die Aufarbeitung von Nebenprodukten, der sog. Überkondensate beschrieben. Bei 120 - 300 °C findet mit einer wässrigen Alkali-Lösung in einer sogenannten Druckdestillationskolonne unter laufender Entfernung des gebildeten Acetons die Hydrolyse der Überkondensate statt.

Die Gewinnung von reinem Isophoron aus Isophoron-haltigen Kondensationsprodukten gelingt durch eine Abtrennung der Leichtsieder durch Destillation unter demselben Druck, bei dem die Kondensation durchgeführt wird und durch eine weitere Aufarbeitung der noch bestehenden Überkondensate durch Destillation bei vermindertem Druck (DE 11 44 269).

Laut der Anmeldung von BP Chemicals lässt sich durch Verwendung von Kalilauge (KOH) anstelle des sonst üblichen Katalysators Natronlauge (NaOH) die Isophoronausbeute bei gleichbleibender Selektivität um bis zu 7 % steigern (DE 25 20 681).

Weiterhin ist beschrieben, dass die Produktqualität des Isophorons erhöht werden kann, indem man farbbildende Substanzen in einem Seitenstrom aus der Reaktionskolonne ausschleust, und diesen Strom durch Destillation und saure Umsetzung aufreinigt (DE 26 45 281).

Weiterhin existieren Anmeldungen zur Isophoron-Herstellung von Daicel Chemical Industries (JP 8245485, JP 8245486) aus den 1990er Jahren. Diese beschreiben, dass durch Erniedrigung der Wasserkonzentration im Eduktstrom als auch durch Rückführung der wässrigen Laugephase nach der Phasentrennung in den Hydrolyseteil der Reaktivdestillation der Isophoronumsatz gesteigert werden kann.

Aus der WO 2012/076314 ist ein Verfahren bekannt zur Herstellung von Isophoron durch katalysierte Aldol-Kondensationen mit Aceton als Edukt,
Aufarbeitung des Reaktionsproduktes,
Hydrolyse des Wertstroms und Trennung in eine organische und eine wässrige Fraktion, Gewinnung von Isophoron aus der organischen Fraktion,
destillative Aufarbeitung der wässrigen Fraktion und Weiterleitung der Brüden vom Kopf der Apparatur der destillativen Aufarbeitung in die Hydrolyse Apparatur.

Bei diesem Verfahren findet die Rückspaltung der Überkondensate in der Hydrolyse aufgrund der Gleichgewichtslagen der stattfindenden Reaktionen nicht vollständig statt, so dass sich in den am Ende der Isophoronaufreinigung anfallenden Rückständen noch hydrolysierbare Überkondensate befinden, die sich durch eine zusätzliche Hydrolyse in Wertstoffe umwandeln lassen. Im bestehenden Verfahren gemäß WO 2012/076314 werden diese Rückstände der Verbrennung zugeführt.

Bei der Synthese von Isophoron entsteht eine ganze Reihe von Nebenprodukten. Diese sind bspw. Diacetonalkohol, Mesityloxid, Phoron, Mesitylen sowie eine Reihe von höheren Kondensationsprodukten (Überkondensate) des Acetons (z. B. Xylitone und Isoxylitone). Aus diesem Grund ist die Erzielung von hohen Ausbeuten und Selektivitäten an Isophoron schwierig zu erreichen.

Die technische Aufgabe dieser Erfindung war es daher, ein Verfahren zu finden, das es ermöglicht, die Wirtschaftlichkeit der Isophoron Herstellung nochmals zu erhöhen.

Gegenstand der Erfindung ist ein Verfahren zur Herstellung von Isophoron durch
1. katalysierte Aldol-Kondensationen von Aceton als Edukt;
2. Aufarbeitung des Reaktionsproduktes,
   wobei die Aufarbeitung der Stufe 2 in drei Fraktionen erfolgt:
   a) Einer Fraktion aus nicht umgesetzten Aceton, Wasser und Leichtsiedern, die kondensiert und anschließend zur Reaktion in den Reaktor zurückgeführt wird;
   b) Einer Fraktion, in der insbesondere farbgebende Substanzen angereichert werden, wobei diese Fraktion weiter aufgereinigt wird und die enthaltenden Wertstoffe im Prozess zurückgeführt werden;
   c) Einer Fraktion aus Isophoron, höherkondensierten Produkten und Wasser und Katalysator, genannt Wertstoffstrom, wobei diese Fraktion anschließend einer Hydrolyse 1 unterworfen wird, bei der Nebenprodukte teilweise oder vollständig in Isophoron, Aceton und andere Wertprodukte überführt werden;
3. wobei die aufgearbeitete Fraktion c) einer Phasentrennung unterworfen wird, in eine im Wesentlichen organische Fraktion d) und eine im Wesentlichen wässrige Fraktion e);
4. destillative Aufarbeitung der wässrigen Fraktion e) und Weiterleitung der Brüden vom Kopf der Apparatur der destillativen Aufarbeitung in die Hydrolyseapparatur 1;
5. Aufreinigung der organischen Fraktion d) in die folgenden drei Fraktionen:
   i) Rückstand,
   ii) Reinisophoron,
   iii) verbliebene Leichtsieder;
6. Hydrolyse 2 des Rückstands i) und Rückführung der entstehenden Wertprodukte in den Prozess.

Überraschenderweise wurde gefunden, dass die Hydrolyse der bei der Isophoronherstellung gebildeten Rückstände effizienter verläuft, wenn man eine weitere Hydrolyse nach der Abtrennung des Isophorons durchgeführt. Die erneute Hydrolyse (Hydrolyse 2) 6., Rückstand i) führt zu einer weiteren Umwandlung der noch vorhandenen hydrolysierbaren Bestandteile des Rückstands. Dies führt zu einer Steigerung der Ausbeute von Isophoron im Hinblick auf die Einsatzmenge von Aceton und zu einer Steigerung der Wirtschaftlichkeit der Isophoronherstellung.

Das erfinderische Verfahren kann kontinuierlich, diskontinuierlich oder semikontinuierlich durchgeführt werden. Es wird jedoch in bevorzugter Weise kontinuierlich durchgeführt.

Die Herstellung von Isophoron 1. erfolgt über katalysierte Aldol-Kondensationen mit Aceton als Edukt. Dabei reagieren im ersten Schritt zwei Aceton-Moleküle über das Zwischenprodukt Diacetonalkohol unter Wasserabspaltung zu Mesityloxid. In einer Folgereaktion reagiert das Mesityloxid mit einem weiteren Aceton wiederum unter Wasserabspaltung zum Isophoron.

Bei Isophoron handelt es sich also um das Reaktionsprodukt einer Kondensation von drei Molekülen Aceton unter der Abspaltung von zwei Molekülen Wasser.

Als Folge der chemischen Ähnlichkeit des eingesetzten Edukts (Aceton) und der gebildeten (Zwischen-) Produkte verläuft die Isophoron-Synthese nicht allzu selektiv. Aufgrund der Vielzahl von konkurrierenden Aldol-Kondensationsreaktionen erhält man unter Reaktionsbedingungen neben dem gewünschten Zielmolekül Isophoron sowohl eine ganze Reihe von unerwünschten (höheren) Kondensationsprodukten (z. B. Xylitone und Isoxylitone) als auch weitere Nebenkomponenten (z. B. Mesitylen).

Die Isophoron-Synthese ist also durch ein komplexes Reaktionsnetzwerk gekennzeichnet; die Selektivität ist in hohem Maße vom Umsatz abhängig. Um die Bildung von unerwünschten (höheren) Kondensationsprodukten zu minimieren, muss der Acetonumsatz begrenzt werden. Insbesondere in der Gasphasenreaktion kann der verwendete Katalysator durch sich bildende Verkokungsrückstände desaktiviert werden.

Es wurde gefunden, dass das entstehende Reaktionsgemisch durch das erfinderische Verfahren besonders wirtschaftlich und ökologisch zu Isophoron aufgearbeitet werden kann.

Die Kondensationsreaktion von Aceton zu Isophoron (Reaktion) wird bevorzugt in einer katalysierten Flüssigphasenreaktion durchgeführt. Alternativ lässt sich Isophoron auch mittels einer Gasphasenreaktion oder auch durch Reaktion in überkritischem Aceton herstellen.

Für die Durchführung der Reaktion gemäß dem erfindungsgemäßen Verfahren in der Flüssigphase wird das Aceton innerhalb des verwendeten Reaktors durch katalytische Reaktion bei Temperaturen im Bereich von 100 bis 250 °C, bevorzugt 150 - 250 °C, besonders bevorzugt 180 - 250 °C und einem Druckbereich von 5 bis 50 bar, bevorzugt 10 - 50 bar, besonders bevorzugt von 20 - 50 bar zu Isophoron umgesetzt, wobei die angegebenen Werte beliebig miteinander kombiniert werden können.

Für die Durchführung der Reaktion gemäß dem erfindungsgemäßen Verfahren in der Gasphase wird das Aceton innerhalb des verwendeten Reaktors durch katalytische Reaktion bei Temperaturen im Bereich von 100 bis 400 °C, bevorzugt 200 - 400 °C zu Isophoron umgesetzt.

Für die Durchführung der Reaktion gemäß dem erfindungsgemäßen Verfahren im überkritischen Bereich wird das Aceton innerhalb des verwendeten Reaktors durch katalytische Reaktion bei Temperaturen im Bereich von 250 bis 350 °C und einem Druckbereich von 50 bis 200 bar zu Isophoron umgesetzt.

Die katalytische Reaktion kann mit den im Stand der Technik genannten Katalysatoren durchgeführt werden, dabei kann es sich entweder um einen homogenen oder einen heterogenen Katalysator handeln. In der Flüssigphase wird bevorzugt ein homogener Katalysator eingesetzt, in der Gasphase bevorzugt ein heterogener Katalysator verwendet. Für die Reaktion im überkritischen Bereich können sowohl homogene als auch heterogene Katalysatoren verwendet werden.

Bei der bevorzugten Reaktion in der Flüssigphase lässt sich Isophoron mittels homogenem Katalysator mit Alkalimengen (NaOH oder KOH) von < 1 Gew.-%, bevorzugt von
< 0,5 Gew.-%, besonders bevorzugt < 0,2 Gew.-%, herstellen. Besonders bevorzugt wird als Katalysator NaOH in Mengen von 0,015 bis 0,05 Gew.-% eingesetzt. Die eingesetzte Wasserkonzentration ergibt sich u. a. aus den Rückführströmen der Aufarbeitungsprozesse, sie sollte bezogen auf die gesamte Flüssigkeitsmenge unterhalb < 40 %, bevorzugt < 30 % liegen.

Die Reaktion kann in beliebigen Reaktoren nach dem Stand der Technik, wie z.B. Rohrreaktoren, Rührkesseln, Rührkesselkaskaden, Festbettreaktoren, Druckdestillationsreaktoren bzw. Reaktivdestillationen, mikrostrukturierten Reaktoren, Schlaufenreaktoren usw. oder in Kombinationen aus beliebigen Reaktoren durchgeführt werden. Dabei ist die Wahl der Reaktoren nicht auf die genannte Auswahl beschränkt.

Der Begriff Druckdestillationsreaktor ist hier gleichzusetzen mit Apparaten, in denen eine Reaktivdestillation durchgeführt wird. Die Reaktivdestillation ist in der Fachliteratur hinlänglich beschrieben, z.B. in Ullmann's Encylcopedia of Industrial Chemistry (M. Sakuth, D. Reusch, R. Janowsky: Reactive Distillation © 2008 Wiley-VCH Verlag GmbH & Co KGaA, Weinheim, DOI: 10.1002/14356007.c22_c01.pub2). Hier und in der zitierten Literatur sind alle gängigen Prozesse und Apparate der Reaktivdestillation beschrieben. Wird im folgenden Text der Patentschrift der Begriff der Reaktivdestillationskolonne verwendet, so sind sämtliche Ausführungsformen der Reaktivdestillation, wie in der Literatur beschrieben, gemeint.

In bevorzugter Ausführung erfolgt die Reaktionsdurchführung in Reaktivdestillationskolonnen, Rohrreaktoren oder Festbettreaktoren. Besonders bevorzugt sind Rohrreaktoren.

Nach Durchführung der Reaktion wird das Reaktionsgemisch in Stufe 2. aufgearbeitet und in die einzelnen Komponenten getrennt. Diese sind neben Isophoron sogenannte Leichtsieder wie z. B. Aceton, Diacetonalkohol und Mesityloxid, sowie eine Reihe von höheren Kondensationsprodukten (Überkondensate) des Acetons (z. B. Xylitone und Isoxylitone), Wasser und gegebenenfalls Katalysator. Dabei wird die Trennung ganz oder teilweise durchgeführt.

Die Abtrennung der einzelnen Fraktionen kann mit allen Trennmethoden wie z. B. Destillation, Entspannungsverdampfung, Kristallisation, Extraktion, Sorption, Permeation, Phasentrennung oder Kombinationen aus den genannten, kontinuierlich oder absatzweise, ein- oder mehrstufig durchgeführt werden. Bevorzugt wird die Trennung durch Destillation in einem oder mehreren Apparaten erreicht.
Dabei kann die Destillation räumlich getrennt von der Isophoronsynthese (Reaktion) durchgeführt werden oder in einem Apparat stattfinden. Bevorzugt erfolgt die Abtrennung der einzelnen Fraktionen durch eine Reaktivdestillation, bevorzugt in einer Reaktivdestillationskolonne.

Besonders bevorzugt wird die Abtrennung räumlich getrennt von der Isophoronsynthese (Reaktion) in einer Reaktivdestillationskolonne mit einer Seitenstromentnahme durchgeführt.

Die Abtrennung erfolgt erfindungsgemäß in drei Fraktionen:
a) Einer Fraktion aus nicht umgesetzten Aceton, Wasser und Leichtsiedern, wie z.B. Diacetonalkohol und Mesityloxid, die kondensiert und anschließend zur Reaktion in den Reaktor zurückgeführt wird.
b) Einer Fraktion, in der insbesondere farbgebende Substanzen angereichert werden. Diese Fraktion wird weiter aufgereinigt und die enthaltenden Wertstoffe werden im Prozess zurückgeführt.
c) Einer Fraktion aus insbesondere Isophoron, höherkondensierten Produkten und Wasser und gegebenenfalls Katalysator, genannt Wertstrom. Diese Fraktion wird anschließend einer Hydrolyse 1 unterworfen.

Die Fraktion a) wird in der bevorzugten Ausführungsform als Brüdenstrom entnommen, enthaltend im Wesentlichen Aceton, Wasser und Leichtsieder, im Wesentlichen Diacetonalkohol und Mesityloxid, kondensiert und dem Reaktor mit den Einsatzstoffen Aceton, Wasser und gegebenenfalls Katalysator wieder zugesetzt.
Die Fraktion b) wird in der bevorzugten Ausführungsform als Seitenstrom der Destillationskolonne, bevorzugt einer Reaktivdestillationskolonne, entnommen, gegebenenfalls neutralisiert und weiter aufgearbeitet. Dabei können bei der Aufarbeitung alle gängigen Trennmethoden wie z.B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich oder absatzweise, ein- oder mehrstufig durchgeführt werden. Bevorzugt wird die Aufreinigung durch Destillation erreicht. Besonders bevorzugt wird die Aufreinigung durch eine Kombination aus Neutralisation oder Extraktion und anschließender Destillation, bevorzugt in einer Reaktivdestillationskolonne, erreicht. Bevorzugt wird die aufgearbeitete Phase mit den Wertprodukten aus Isophoron, Hochsiedern und gegebenenfalls Katalysator in die Hydrolyse geführt. Eine weitere erhaltene Phase aus Wertprodukten, im Wesentlichen enthaltend Aceton, Diacetonalkohol und Mesityloxid, wird bevorzugt in die Reaktion zurückgeführt. Gegebenenfalls anfallende Rückstände werden der thermischen Verwertung zugeführt.

Die Fraktion c) wird einer Hydrolyse 1 unterworfen. Das Ziel der Hydrolyse 1 ist es, Nebenprodukte teilweise oder vollständig in Isophoron, Aceton und andere Wertprodukte zu überführen. Die Hydrolyse kann in allen gängigen Reaktoren, welche bereits oben beschrieben wurden, oder Destillationskolonnen oder Kombinationen aus beiden, durchgeführt werden. Bevorzugt wird die Hydrolyse durch eine Reaktivdestillation durchgeführt, in dem die entstehenden Leichtsieder, im Wesentlichen enthaltend Aceton, Diacetonalkohol und Mesityloxid, direkt aus der Hydrolysezone entfernt und in die Reaktion zurückgeführt werden, und somit nicht mehr für Nebenreaktionen in der Hydrolyse zur Verfügung stehen.

Ganz besonders bevorzugt wird die Hydrolyse 1 der Fraktion c) in einem Apparat, durch eine Reaktivdestillation durchgeführt, bevorzugt in einer Reaktivdestillationskolonne, wobei gleichzeitig die Trennung des Reaktionsgemisches in die Fraktionen a) bis c) erfolgt, so dass die entstehenden Produkte gleichzeitig entsprechend aufgetrennt werden und die Fraktion c) hydrolysiert wird.

Gegebenenfalls können die Hydrolyse 1 und die destillative Abtrennung auch in einem Apparat mit der Isophoronsynthese (Reaktion) stattfinden.

Die Hydrolyse 1 kann in allen Mischungsverhältnissen der organischen Komponenten mit Wasser mit oder ohne Katalysator durchgeführt werden. Die Wasserkonzentration in der Hydrolyse beträgt dabei 0,1 - 99,9 Gew.-%, bevorzugt 30 - 90 Gew.-%. Im Falle der homogenen Katalyse wird bei der Hydrolyse bevorzugt derjenige Katalysator eingesetzt, der auch im Reaktionsteil verwendet wird. Bevorzugt werden Katalysatorkonzentrationen von 0,001 - 10 Gew.-%, besonders bevorzugt von 0,05 - 1 Gew.-%. Der Druck im Hydrolysereaktor beträgt 1-200 bar, bevorzugt 20 - 60 bar, besonders bevorzugt wird die Hydrolyse mindestens bei dem Druck durchgeführt, der auch im Isophoronsyntheseschritt (Reaktion) herrscht. Die Temperatur der Hydrolyse beträgt 100 - 300 °C, bevorzugt 210 - 260 °C. Besonders bevorzugt wird sich bei Verwendung einer Reaktivdestillationskolonne eine Temperatur bzw. ein Temperaturverlauf einstellen, der den Siedetemperaturen im Sumpf und auf den einzelnen Trenn- oder Reaktionsstufen entspricht.

Die Hydrolyse 1 kann in einem oder mehreren Apparaten einstufig oder mehrstufig durchgeführt werden.

Die so aufgearbeitete Fraktion c) wird anschließend aus dem Hydrolysereaktor 1 bzw. der Reaktivdestillationskolonne abgetrennt, abgekühlt und einer Phasentrennung unterworfen.

Die Phasentrennung erfolgt in eine im Wesentlichen organische Fraktion d) und eine im Wesentlichen wässrige Fraktion e), die, bei homogener Katalyse, auch den Katalysator enthält. Dabei können übliche Phasentrennbehälter mit und ohne Einbauten zum Einsatz kommen. Die Phasentrennung erfolgt bei einer Temperatur zwischen 0 - 200 °C, bevorzugt bei 0 - 100 °C, besonders bevorzugt bei 20 - 70 °C und einem Druck von 1 - 150 bar, bevorzugt 20 - 60 bar, besonders bevorzugt bei dem Druck, der auch in der Hydrolyse herrscht.

Die im Wesentlichen organische Fraktion d) mit dem Zielprodukt Isophoron wird gegebenenfalls neutralisiert und mit üblichen Methoden aufgereinigt, so dass ein Isophoron mit der gewünschten Reinheit und Farbstabilität erhalten wird. Dabei können alle gängigen Trennmethoden wie z.B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich oder absatzweise, ein- oder mehrstufig, unter Druck oder im Vakuum durchgeführt werden.
Die Aufreinigung der organischen Fraktion d) erfolgt in die folgenden drei Fraktionen:
i) Rückstand,
ii) Reinisophoron
iii) verbliebene Leichtsieder.

Bevorzugt wird die Aufreinigung durch Destillation erreicht. Besonders bevorzugt wird die Aufreinigung durch eine Kombination aus Neutralisation oder Extraktion und folgender Destillation erreicht.

Die Fraktion i) Rückstand wird einer zusätzlichen Hydrolyse 6. (Hydrolyse 2) unterzogen.

Die Hydrolyse 2 (6.) erfolgt unter denselben Bedingungen wie die Hydrolyse 1. Die Hydrolyse 2 kann in allen Mischungsverhältnissen der organischen Komponenten mit Wasser mit oder ohne Katalysator durchgeführt werden. Die Wasserkonzentration in der Hydrolyse beträgt dabei 0,1 - 99,9 Gew.-%, bevorzugt 30 - 90 Gew.-%. Im Falle der homogenen Katalyse wird bei der Hydrolyse bevorzugt derjenige Katalysator eingesetzt, der auch im Reaktionsteil verwendet wird. Bevorzugt werden Katalysatorkonzentrationen von 0,001 - 10 Gew.-%, besonders bevorzugt von 0,05 - 1 Gew.-%. Der Druck im Hydrolysereaktor beträgt 1-200 bar, bevorzugt 20 - 60 bar, die Temperatur der Hydrolyse beträgt 100 - 300 °C, bevorzugt 210 - 260 °C.

Die Hydrolyse 2 kann in einem oder mehreren Apparaten einstufig oder mehrstufig durchgeführt werden.

Die Hydrolyse 2 kann in allen gängigen Reaktoren, welche bereits oben beschrieben wurden, oder Destillationskolonnen oder Kombinationen aus beiden, bevorzugt in mindestens einer Reaktivdestillationkolonne, durchgeführt werden.

Zu den Wertprodukten in den Brüden, die bei der Hydrolyse 2 des Rückstands i) entstehen, gehören neben Isophoron und Aceton weitere zu Isophoron umsetzbare Leichtsieder, insbesondere Diacetonalkohol, Mesityloxid und Phoron. Die entstehenden Wertprodukte werden anschließend in den Prozess zurückgeführt und somit stofflich verwertet. Bevorzugt wird in die Hydrolyse 1 zurückgeführt. Bevorzugt erfolgt die Weiterleitung der Brüden vom Kopf der Hydrolyseapparatur 2 in die Hydrolyseapparatur 1. Besonders bevorzugt erfolgt die Hydrolyse 2 einstufig in einer Reaktivdestillationkolonne.

Durch die Hydrolyse 6. des Rückstands i) ergeben sich Vorteile im Vergleich zum Prozess der WO 2012/076314. Der Rückstand i), der der Hydrolyse 2 zugeführt wird, beinhaltet nahezu kein Isophoron, dies führt zu einer günstigeren Lage des Reaktionsgleichgewichts als bei der Hydrolyse der Fraktion c) (Hydrolyse 1), so dass die Hydrolyse vollständiger ablaufen kann.

An dieser Stelle wird die destillative Aufarbeitung der wässrigen Fraktion e) (Abwasseraufreinigung) und Weiterleitung der Brüden vom Kopf der Apparatur der destillativen Aufbereitung in die Hydrolyseapparatur 1 genauer beschrieben.

Die im Wesentlichen wässrige Fraktion e) wird bevorzugt einer Abwasseraufreinigung zugeführt. Hier erfolgt die Trennung des Reaktionswassers als Hauptbestandteil und gegebenenfalls des Katalysators von gegebenenfalls noch gelösten organischen Komponenten, wie z. B. Isophoron, Aceton und höher kondensierten Produkten. Die Abwasseraufreinigung wird in der WO 2012/076314 und WO 2012 156187 genau beschrieben.

Bevorzugt wird die Abwasseraufreinigung in Gegenwart eines Entschäumers durchgeführt, wodurch die Schaumbildung im Sumpf dieser Abwasserkolonne nahezu vollständig verhindert wird und somit eine sehr gute Trennung zwischen dem Hauptbestandteil Wasser und den noch gelösten organischen Komponenten, wie z. B. Isophoron, Aceton und andere Leichtsieder, sowie höhere siedende Produkte, erzielt wird. Außerdem lässt sich somit die Füllstandshöhe der Abwasserkolonne exakt bestimmen, und sowohl ein Überlaufen als auch ein Trockenlaufen der Abwasserkolonne wird verhindert.

Die Abwasseraufreinigung wird bevorzugt in einer oder mehreren Destillationskolonnen durchgeführt. Erfindungswesentlich ist dabei, dass die Brüden der Abwasserkolonne direkt in den Apparat geleitet werden, in dem die Hydrolyse 1 stattfindet. Dadurch werden mehrere Probleme des derzeitigen Standes der Technik gleichzeitig gelöst:
1) Da die Brüden im Wesentlichen aus Wasser bestehen, stellt sich im Hydrolyseteil eine notwendige, ausreichend hohe Wasserkonzentration ein, so dass kein zusätzliches Frischwasser in die Hydrolyse eingebracht werden muss.
2) Die in der Fraktion e) gelösten, organischen Anteile werden teilweise oder komplett über die Brüden der Abwasserkolonne in den Prozess zurückgeführt. Das minimiert die organische Belastung im Abwasser und, da es sich im Wesentlichen um Isophoron handelt, erhöht die Gesamtausbeute im Prozess. Diese neuartige Verschaltung der Abwasserkolonne trägt somit einen wesentlichen Beitrag zur ökologischen und ökonomischen Prozessführung bei.
3) Außerdem wird die notwendige Wärme für die Hydrolyse bzw. die destillative Trennung des Reaktionsgemisches durch die Brüden zur Verfügung gestellt, es wird keine separate Heizung benötigt.

Der Druck in der Abwasserkolonne beträgt 1 - 200 bar, bevorzugt 20 - 60 bar. Besonders bevorzugt wird bei dem Systemdruck gearbeitet, der sich im Gesamtsystem Hydrolyse/Abwasserkolonne einstellt, wenn die Brüden der Abwasserkolonne direkt in den Hydrolyseteil der Reaktivdestillation geleitet werden. Die Temperatur in der Abwasserkolonne entspricht der Siedetemperatur der Fraktion e) unter den Druckbedingungen. Die bevorzugte Temperatur der Brüden beträgt 200 - 300 °C.

Im Folgenden wird näher beschrieben, wie das Wasser aus dem Sumpf der destillativen Aufarbeitung der wässrigen Fraktion e) einer Entspannungsverdampfung unterworfen wird und das entstehende gereinigte Wasser in den Prozess zur Herstellung von Isophoron zurückgeführt wird.

Das im Sumpf der Abwasserkolonne anfallende Abwasser (Strom f) kann abgekühlt und verworfen werden. Bevorzugt wird das Abwasser f) aber einer Entspannungsverdampfung zugeführt und somit weiter aufgetrennt. Die Brüden g) der Entspannungverdampfungsstufe, die im Wesentlichen aus reinem Wasser bestehen, können kondensiert und als Wasser in den Prozess, bevorzugt in die Reaktion, z. B. zur Verdünnung des eingesetzten Katalysators (im Falle der homogenen Katalyse) zurückgeführt werden. Dadurch wird die Abwassermenge nochmals reduziert. Die Entspannungsverdampfung kann ein- oder mehrstufig kontinuierlich oder diskontinuierlich durchgeführt werden. Der Druck in der Entspannungsverdampfung liegt in jedem Fall unterhalb des Druckes in der Abwasserkolonne. Bevorzugt ist bei dem erfindungsgemäßen Verfahren die Anwendung einer Entspannungsverdampfung.

Sämtliche Destillations- und Reaktionsschritte im Prozess können in Reaktoren oder Apparaten mit oder ohne Einbauten durchgeführt werden, wie z. B. Dephlegmatoren, ungeordnete Einbauten oder Schüttungen, geordnete Einbauten oder Packungen, Böden mit oder ohne Zwangsführung.

Alle für die Reaktion eingesetzten produktberührten metallischen Werkstoffe und die aus den metallischen Werkstoffen hergestellten Apparate sowie deren Einbauten müssen gegen Laugen beständig sein. Dabei können in Abhängigkeit von der Gefährdung unterschiedliche Beständigkeitsanforderungen bestehen. Für die Beständigkeiten sind nicht nur die chemischen und/oder mechanischen Eigenschaften von Bedeutung, sondern auch die zur Anwendung kommenden Fertigungsmethoden und die Beurteilungsmaßstäbe während der Prüfung.

Für die metallischen Werkstoffe wird zum Teil Bezug auf das AD 2000-Merkblatt HP 0 Ausgabe 11.2008 (Allgemeine Grundsätze für Auslegung, Herstellung und damit verbundene Prüfungen) und DIN EN 10020 Ausgabe 07.2000 (Begriffsbestimmung für die Einteilung der Stähle) genommen. Die dort genannten Werkstoffgruppen werden zitiert, um die Bezeichnungen (z. B. "austenitscher nichtrostender Stahl) zu präzisieren. Sofern technisch sinnvoll, gelten die Aussagen für alle technisch verfügbaren Varianten der Werkstoffe (z. B. Schmiedevarianten, Walzvarianten und Gussvarianten) mit vergleichbarer Beständigkeit gegen Laugekorrosion.
a) Für drucktragende und produktberührte Komponenten können beliebige, nach dem Stand der Technik geeignete Werkstoffe zur Anwendung kommen wie z. B.:
   - Warmfeste Stähle (z. B. Werkstoffuntergruppe 5.1 bis 5.4 und 6.1 bis 6.4 gemäß AD 2000 HP 0)
   - Austenitische nichtrostende Stähle (z. B. Werkstoffuntergruppe 8.1 bis 8.2 gemäß AD 2000 HP 0)
   - Ferritfreie austenitische nichtrostende Stähle (z. B. Werkstoffuntergruppe 8.1 bis 8.2 gemäß AD 2000 HP 0)
   - Ferritisch-austenitische nichtrostende Stähle (z. B. Werkstoffuntergruppe 10.1 bis 10.2 gemäß AD 2000 HP 0)
   - Nickel und Nickellegierungen (z. B. Werkstoffuntergruppe 41 bis 46 gemäß AD 2000 HP 0)

Auch Kombinationen der o. g. Werkstoffe können zur Anwendung kommen. Dabei ist die Wahl der Werkstoffe nicht auf die genannte Auswahl beschränkt und umfasst auch korrosionstechnisch gleichwertige oder höherwertige Varianten. Bevorzugt werden Werkstoffe, die sich nach dem Stand der Technik unter Berücksichtigung der Beanspruchungsbedingungen und Gefährdungen durch eine technische Beständigkeit gegen Laugen auszeichnen. Ein Verzicht auf Wärmebehandlungen ist nicht möglich, wenn sich dadurch die technische Beständigkeit gegen Laugen in unzulässiger Weise ändert.
b) Für nicht drucktragende und produktberührte Komponenten können beliebige, nach dem Stand der Technik geeignete Werkstoffe zur Anwendung kommen wie z. B.:
   - Alle unter a) genannten Werkstoffe
   - Unlegierte Stähle (z. B. Werkstoffuntergruppe 1.1 bis 1.2 gemäß AD 2000 HP 0)
   - Unlegierte Stähle und andere legierte Stähle (z. B. gemäß DIN EN 10020)

Auch Kombinationen der o. g. Werkstoffe können zur Anwendung kommen. Dabei ist die Wahl der Werkstoffe nicht auf die genannte Auswahl beschränkt und umfasst auch korrosionstechnisch gleichwertige oder höherwertige Varianten. Bevorzugt werden Werkstoffe, die sich nach dem Stand der Technik unter Berücksichtigung der Beanspruchungsbedingungen und Gefährdungen durch eine ausreichende Beständigkeit gegen Laugen auszeichnen. Für nicht drucktragende Komponenten können in Abhängigkeit von der Gefährdung gegebenenfalls temporäre Beständigkeiten in Kauf genommen werden. Ein Verzicht auf Wärmebehandlungen ist nicht möglich, wenn sich dadurch die technische Beständigkeit gegen Laugen in unzulässiger Weise ändert.
c) Die Werkstoffeigenschaften werden durch geeignete Fertigungsverfahren verändert, die im Folgenden nach den in der DIN 8580 Ausgabe 09.2003 (Fertigungsverfahren - Begriffe, Einteilung) genannten Bezeichnungen beschrieben sind. Folgende Fertigungsverfahren können für die Verarbeitung der metallischen Werkstoffe z. B. zur Anwendung kommen:
   - Urformen (z. B. Gießen)
   - Umformen (z. B. Kaltumformen und Warmumformen)
   - Trennen (z. B. Spanen mit geometrisch bestimmter Schneide und Spanen mit geometrisch unbestimmter Schneide)
   - Fügen (z. B. Schmelzschweißen)
   - Beschichten (z. B. Beschichten aus dem flüssigen Zustand, Schmelztauchen, Plattieren, Thermisches Spritzen, Sintern, galvanisches Besichten, chemisches Beschichten und Beschichten aus dem gasförmigen und dampfförmigen Zustand)
   - Stoffeigenschaften ändernde Fertigungsverfahren (Verfestigung durch Umformen wie z. B. Schmieden, Walzen, Strahlen; Wärmebehandeln wie z. B. Vergüten, Rekristallisationsglühen, Spannungsarmglühen, Normalisierungsglühen; Thermomechanische Behandlungen wie z. B. die Kombination von Wärmebehandlung und Umformbehandlung; Sintern und Brennen)

Auch Kombinationen der o. g. Fertigungsverfahren können zur Anwendung kommen. Dabei ist die Wahl der Fertigungsverfahren nicht auf die genannte Auswahl beschränkt. Bevorzugt werden Verfahren, die nach dem Stand der Technik die erforderliche Laugenbeständigkeit der jeweiligen Werkstoffe und Apparate gewährleisten.
d) Folgende Prüfungen an Apparaten und Einbauten sowie insbesondere an deren Schweißverbindungen können beispielsweise zur Anwendung kommen:
   - Magnetpulverprüfung MT
   - Eindringprüfung PT
   - Durchstrahlungsprüfung RT
   - Ultraschallprüfung UT
   - Sichtprüfung VT
   - Härteprüfung HT
   - Legierungsanalyse

Auch Kombinationen der o. g. Prüfverfahren sind möglich. Dabei ist die Wahl der Prüfverfahren nicht auf die genannte Auswahl beschränkt. Es werden Prüfverfahren und Bewertungsgrundlagen bevorzugt, die nach dem Stand der Technik dazu beitragen, die erforderliche Laugenbeständigkeit der jeweiligen Komponenten zu gewährleisten.

### Beispiel erfindungsgemäß:

Die Herstellung von Isophoron erfolgte gemäß den Schritten, wie in der Beschreibung beschrieben. Die Fraktion d) wurde destillativ aufgearbeitet. Es fallen 0.12 kg Rückstand i) pro produziertem kg Isophoron an.
Der Rückstand i) aus der Aufarbeitung wurde einer zusätzlichen Hydrolyse 2 unterzogen.

Die Hydrolyse 2 wurde in einer einstufigen Destillationsapparatur nach dem Prinzip einer Reaktivdestillation durchgeführt. Diese Apparatur besteht aus einem beheizten Rührbehälter sowie zwei Leitungen um Wasserdampf bzw. Stickstoff in den Behälter zu leiten. Außerdem gibt es einen Anschluss um die entstehenden Brüden abzuführen, zu kondensieren und aufzufangen. Das Experiment wurde unter den folgenden Bedingungen durchgeführt: 300 g Rückstand i) und 1500 g Wasser wurden im Reaktor vorgelegt und unter Rühren (ca. 400 U/min) aufgeheizt. Dabei wurden der Stickstoffstrom auf 10 g/h und der Anlagendruck auf 36 bar eingestellt. Nach dem Aufheizen des Reaktors auf 240 °C wurde die 25 %-ige Natronlauge über die Druckvorlage in den Reaktor gedrückt und die Druckvorlage mit der 188 g Wasser gespült. Aus dem Rückstand i) wurden 23 Gew.-% Wertstoffe erhalten. Zu den entstehenden Wertstoffen gehören Aceton, Isophoron, Phoron, Diacetonalkohol sowie Mesityloxid.

Vergleich zur Herstellung gemäß WO 2012/076314
Durch die oben gezeigte zusätzliche Hydrolyse können somit pro hergestelltem Kilogramm Isophoron 0.04 kg Aceton im gesamten Prozess gemäß den Stufen 1-6 eingespart werden. Die Ausbeute an Isophoron wurde um 3 Gew.-% im gesamten Prozess gemäß den Stufen 1-6 gesteigert.

## Patentansprüche

1. Verfahren zur Herstellung von Isophoron durch
1. katalysierte Aldol-Kondensationen von Aceton als Edukt;
2. Aufarbeitung des Reaktionsproduktes,
wobei die Aufarbeitung der Stufe 2 in drei Fraktionen erfolgt:
a) Einer Fraktion aus nicht umgesetzten Aceton, Wasser und Leichtsiedern, die kondensiert und anschließend zur Reaktion in den Reaktor zurückgeführt wird;
b) Einer Fraktion, in der insbesondere farbgebende Substanzen angereichert werden, wobei diese Fraktion weiter aufgereinigt wird und die enthaltenden Wertstoffe im Prozess zurückgeführt werden;
c) Einer Fraktion aus Isophoron, höherkondensierten Produkten und Wasser und Katalysator, genannt Wertstoffstrom, wobei diese Fraktion anschließend einer Hydrolyse 1 unterworfen wird, bei der Nebenprodukte teilweise oder vollständig in Isophoron, Aceton und andere Wertprodukte überführt werden;
3. wobei die aufgearbeitete Fraktion c) einer Phasentrennung unterworfen wird, in eine imWesentlichen organische Fraktion d) und eine im Wesentlichen wässrige Fraktion e);
4. destillative Aufarbeitung der wässrigen Fraktion e) und Weiterleitung der Brüden vom Kopf der Apparatur der destillativen Aufarbeitung in die Hydrolyseapparatur 1;
5. Aufreinigung der organischen Fraktion d) in die folgenden drei Fraktionen:
i) Rückstand,
ii) Reinisophoron,
iii) verbliebene Leichtsieder;
6. Hydrolyse 2 des Rückstands i) und Rückführung der entstehenden Wertprodukte in den Prozess.

2. Verfahren zur Herstellung von Isophoron nach Anspruch 1, **dadurch gekennzeichnet, dass** das Wasser aus dem Sumpf der destillativen Aufarbeitung der wässrigen Fraktion e) einer Entspannungsverdampfung unterworfen wird und das entstehende gereinigte Wasser in den Prozess zur Herstellung von Isophoron zurückgeführt wird.

3. Verfahren zur Herstellung von Isophoron nach Anspruch 1 oder 2, **dadurch gekennzeichnet, dass** die Durchführung der Reaktion in der Flüssigphase erfolgt, wobei das Aceton durch katalytische Reaktion bei Temperaturen im Bereich von 100 bis 250 °C, bevorzugt 150 - 250 °C, besonders bevorzugt 180 - 250 °C und einem Druckbereich von 5 bis 50 bar, bevorzugt 10 - 50 bar, besonders bevorzugt von 20 - 50 bar zu Isophoron umgesetzt wird, wobei die angegebenen Werte beliebig miteinander kombiniert werden können.

4. Verfahren zur Herstellung von Isophoron nach Anspruch 1 oder 2, **dadurch gekennzeichnet, dass** die Durchführung der Reaktion in der Gasphase erfolgt, wobei das Aceton durch katalytische Reaktion bei Temperaturen im Bereich von 100 bis 400 °C, bevorzugt 200 - 400 °C zu Isophoron umgesetzt wird.

5. Verfahren zur Herstellung von Isophoron nach Anspruch 1 oder 2, **dadurch gekennzeichnet, dass** die Durchführung im Überkritischen Bereich erfolgt, wobei das Aceton durch katalytische Reaktion bei Temperaturen im Bereich von 250 bis 350 °C und einem Druckbereich von 50 bis 200 bar zu Isophoron umgesetzt wird.

6. Verfahren zur Herstellung von Isophoron nach mindestens einem der vorherigen Ansprüche, **dadurch gekennzeichnet, dass** ein homogener oder ein heterogener Katalysator, bevorzugt ein homogener Katalysator eingesetzt wird.

7. Verfahren zur Herstellung von Isophoron nach mindestens einem der vorherigen Ansprüche 1 - 3, **dadurch gekennzeichnet, dass** die Reaktion in der Flüssigphase mit einem homogenen Katalysator erfolgt.

8. Verfahren zur Herstellung von Isophoron nach Anspruch 7, **dadurch gekennzeichnet, dass** Alkalimengen von < 1 Gew.-%, bevorzugt von < 0,2 Gew.-% als Katalysator, bevorzugt NaOH in Mengen von 0,015 bis 0,05 Gew.-% eingesetzt werden

9. Verfahren zur Herstellung von Isophoron nach mindestens einem der vorherigen Ansprüche, **dadurch gekennzeichnet, dass** die Reaktion in Reaktoren ausgewählt aus Rohrreaktoren, Rührkesseln, Rührkesselkaskaden, Festbettreaktoren, Reaktivdestillationskolonnen, mikrostrukturierten Reaktoren, Schlaufenreaktoren oder in Kombinationen aus diesen Reaktoren, durchgeführt wird.

10. Verfahren zur Herstellung von Isophoron nach mindestens einem der vorherigen Ansprüche, **dadurch gekennzeichnet, dass** die Reaktion in Reaktivdestillationskolonnen, Rohrreaktoren oder Festbettreaktoren, besonders bevorzugt in einem Rohrreaktor, durchgeführt wird.

11. Verfahren zur Herstellung von Isophoron nach mindestens einem der vorherigen Ansprüche, **dadurch gekennzeichnet, dass** die Abtrennung der einzelnen Fraktionen in Stufe 2. durch Trennmethoden wie Destillation, Entspannungsverdampfung, Kristallisation, Extraktion, Sorption, Permeation, Phasentrennung oder Kombinationen aus den genannten, kontinuierlich oder absatzweise, ein- oder mehrstufig durchgeführt wird, bevorzugt durch Destillation in einem oder mehreren Apparaten.

12. Verfahren zur Herstellung von Isophoron nach mindestens einem der vorherigen Ansprüche, **dadurch gekennzeichnet, dass** die Abtrennung in Stufe 2 durch Destillation räumlich getrennt von der Isophoronsynthese durchgeführt wird oder in einem Reaktionsapparat stattfindet, bevorzugt durch eine Reaktivdestillation, bevorzugt in einer Reaktivdestillationskolonne.

13. Verfahren zur Herstellung von Isophoron nach mindestens einem der vorherigen Ansprüche, **dadurch gekennzeichnet, dass** die Abtrennung in Stufe 2 räumlich getrennt von der Isophoronsynthese durch eine Reaktivdestillation, bevorzugt in einer Reaktivdestillationskolonne, mit einer Seitenstromentnahme durchgeführt wird.

14. Verfahren zur Herstellung von Isophoron nach mindestens einem der vorherigen Ansprüche, **dadurch gekennzeichnet, dass** die Fraktion a) als Brüdenstrom entnommen wird, enthaltend im Wesentlichen Aceton, Wasser und Leichtsiedern, im Wesentlichen Diacetonalkohol und Mesityloxid, kondensiert und dem Reaktor mit den Einsatzstoffen Aceton, Wasser und Katalysator wieder zugesetzt wird.

15. Verfahren zur Herstellung von Isophoron nach mindestens einem der vorherigen Ansprüche, **dadurch gekennzeichnet, dass** die Fraktion b) als Seitenstrom einer Destillationskolonne, bevorzugt einer Reaktivdestillationskolonne, entnommen wird.

16. Verfahren zur Herstellung von Isophoron nach mindestens einem der vorherigen Ansprüche, **dadurch gekennzeichnet, dass** die Aufreinigung der Fraktion b) durch eine Kombination aus Neutralisation oder Extraktion und anschließender Destillation in einer Reaktivdestillationskolonne, erfolgt.

17. Verfahren zur Herstellung von Isophoron nach mindestens einem der vorherigen Ansprüche, **dadurch gekennzeichnet, dass** die aufgearbeitete Phase der Fraktion b) mit den Wertprodukten aus Isophoron, Hochsiedern und gegebenenfalls Katalysator in die Hydrolyse geführt wird.

18. Verfahren zur Herstellung von Isophoron nach mindestens einem der vorherigen Ansprüche, **dadurch gekennzeichnet, dass** eine weitere erhaltene Phase der Fraktion b) aus Wertprodukten, im Wesentlichen enthaltend Aceton, Diacetonalkohol und Mesityloxid, in die Reaktion zurückgeführt wird.

19. Verfahren zur Herstellung von Isophoron nach mindestens einem der vorherigen Ansprüche, **dadurch gekennzeichnet, dass** die Hydrolyse c) in einer Reaktivdestillationskolonne erfolgt.

20. Verfahren zur Herstellung von Isophoron nach mindestens einem der vorherigen Ansprüche, **dadurch gekennzeichnet, dass** die Hydrolyse c) bei Wasserkonzentration in der Hydrolyse beträgt dabei 0,1 - 99,9 Gew.-%, bevorzugt 30 - 90 Gew.-%, erfolgt.

21. Verfahren zur Herstellung von Isophoron nach mindestens einem der vorherigen Ansprüche, **dadurch gekennzeichnet, dass** die Hydrolyse c) bei Katalysatorkonzentrationen von 0,001 - 10 Gew.-%, besonders bevorzugt von 0,05 - 1 Gew.-%, erfolgt.

22. Verfahren zur Herstellung von Isophoron nach mindestens einem der vorherigen Ansprüche, **dadurch gekennzeichnet, dass** die Hydrolyse c) bei einem Druck im Hydrolysereaktor von 1 - 200 bar, bevorzugt 20 - 60 bar, besonders bevorzugt mindestens bei dem Druck, der auch im Isophoronsyntheseschritt (Reaktion) herrscht, durchgeführt wird.

23. Verfahren zur Herstellung von Isophoron nach mindestens einem der vorherigen Ansprüche, **dadurch gekennzeichnet, dass** die Hydrolyse c) bei einer Temperatur von 100 - 300 °C, bevorzugt 210 - 260 °C durchgeführt wird.

24. Verfahren zur Herstellung von Isophoron nach mindestens einem der vorherigen Ansprüche, **dadurch gekennzeichnet, dass** die Aufreinigung der organischen Fraktion d) durch Destillation, bevorzugt durch eine Kombination aus Neutralisation oder Extraktion und folgender Destillation, erfolgt.

25. Verfahren zur Herstellung von Isophoron nach mindestens einem der vorherigen Ansprüche, **dadurch gekennzeichnet, dass** die Hydrolyse 6. bei Wasserkonzentration in der Hydrolyse beträgt dabei 0,1 - 99,9 Gew.-%, bevorzugt 30 - 90 Gew.-%, erfolgt.

26. Verfahren zur Herstellung von Isophoron nach mindestens einem der vorherigen Ansprüche, **dadurch gekennzeichnet, dass** die Hydrolyse 6. bei Katalysatorkonzentrationen von 0,001 - 10 Gew.-%, besonders bevorzugt von 0,05 - 1 Gew.-%, erfolgt.

27. Verfahren zur Herstellung von Isophoron nach mindestens einem der vorherigen Ansprüche, **dadurch gekennzeichnet, dass** die Hydrolyse 6. bei einem Druck im Hydrolysereaktor von 1 - 200 bar, bevorzugt 20 - 60 bar, durchgeführt wird.

28. Verfahren zur Herstellung von Isophoron nach mindestens einem der vorherigen Ansprüche, **dadurch gekennzeichnet, dass** die Hydrolyse 6. bei einer Temperatur von 100 - 300 °C, bevorzugt 210 - 260 °C durchgeführt wird.

29. Verfahren zur Herstellung von Isophoron nach mindestens einem der vorherigen Ansprüche, **dadurch gekennzeichnet, dass** die Hydrolyse 6. in einem oder mehreren Apparaten einstufig oder mehrstufig durchgeführt wird.

30. Verfahren zur Herstellung von Isophoron nach mindestens einem der vorherigen Ansprüche, **dadurch gekennzeichnet, dass** die Weiterleitung der Brüden vom Kopf der Hydrolyseapparatur 6. in die Hydrolyseapparatur 1 erfolgt.

31. Verfahren zur Herstellung von Isophoron nach mindestens einem der vorherigen Ansprüche, **dadurch gekennzeichnet, dass** die Hydrolyse 6. in mindestens einer Reaktivdestillationskolonne erfolgt.

32. Verfahren zur Herstellung von Isophoron nach mindestens einem der vorherigen Ansprüche, **dadurch gekennzeichnet, dass** die die Hydrolyse 6. einstufig in einer Reaktivdestillationkolonne erfolgt.

33. Verfahren zur Herstellung von Isophoron nach mindestens einem der vorherigen Ansprüche, **dadurch gekennzeichnet, dass** eine destillative Aufarbeitung der wässrige Fraktion e) erfolgt und Weiterleitung der Brüden vom Kopf der Apparatur der destillativen Aufbereitung in die Hydrolyseapparatur 1, wobei die destillative Aufarbeitung bevorzugt in einer oder mehreren Destillationskolonnen durchgeführt wird.

34. Verfahren zur Herstellung von Isophoron nach Anspruch 33, **dadurch gekennzeichnet, dass** das im Sumpf anfallende Abwasser (Strom f) einer Entspannungsverdampfung zugeführt und weiter aufgetrennt wird.

## Claims

1. Process for preparing isophorone by
1. catalysed aldol condensations with acetone as a reactant;
2. workup of the reaction product,
the workup in stage 2 being effected so as to give three fractions:
a) a fraction composed of unconverted acetone, water and low boilers, which is condensed and then recycled into the reactor for reaction;
b) a fraction in which colouring substances in particular are enriched, this fraction being purified further and the materials of value present being recycled into the process;
c) a fraction composed of isophorone, more highly condensed products and water and catalyst, called material of value stream, this fraction subsequently being subjected to a hydrolysis 1 in which by-products are converted partly or fully to isophorone, acetone and other products of value;
3. wherein the worked-up fraction c) is subjected to a phase separation into an essentially organic fraction d) and an essentially aqueous fraction e) ;
4. distillative workup of the aqueous fraction e) and passage of the vapours from the top of the distillative workup apparatus onward into the hydrolysis apparatus 1;
5. purification of the organic fraction d) to give the following three fractions:
i) residue,
ii) pure isophorone,
iii) remaining low boilers;
6. hydrolysis 2 of the residue i) and recycling of the products of value formed into the process.

2. Process for preparing isophorone according to Claim 1, **characterized in that** the water from the bottoms of the distillative workup of the aqueous fraction e) is subjected to a flash evaporation and the purified water which forms is recycled into the process for preparing isophorone.

3. Process for preparing isophorone according to Claim 1 or 2, **characterized in that** the reaction is performed in the liquid phase, the acetone being converted to isophorone by catalytic reaction at temperatures in the range from 100 to 250°C, preferably 150-250°C and more preferably 180-250°C, and a pressure range of 5 to 50 bar, preferably 10-50 bar and more preferably of 20-50 bar, it being possible to combine the values specified as desired.

4. Process for preparing isophorone according to Claim 1 or 2, **characterized in that** the reaction is performed in the gas phase, the acetone being converted to isophorone by catalytic reaction at temperatures in the range from 100 to 400°C and preferably 200-400°C.

5. Process for preparing isophorone according to Claim 1 or 2, **characterized in that** the procedure is effected in the supercritical range, the acetone being converted to isophorone by catalytic reaction at temperatures in the range from 250 to 350°C and a pressure range of 50 to 200 bar.

6. Process for preparing isophorone according to at least one of the preceding claims, **characterized in that** a homogeneous or heterogeneous catalyst, preferably a homogeneous catalyst, is used.

7. Process for preparing isophorone according to at least one of the preceding Claims 1 - 3, **characterized in that** the reaction is effected in the liquid phase with a homogeneous catalyst.

8. Process for preparing isophorone according to Claim 7, **characterized in that** amounts of alkali of < 1% by weight, preferably of < 0.2% by weight, are used as a catalyst, preferably NaOH in amounts of 0.015 to 0.05% by weight.

9. Process for preparing isophorone according to at least one of the preceding claims, **characterized in that** the reaction is performed in reactors selected from tubular reactors, stirred tanks, stirred tank cascades, fixed bed reactors, reactive distillation columns, microstructured reactors, loop reactors, or in combinations of these reactors.

10. Process for preparing isophorone according to at least one of the preceding claims, **characterized in that** the reaction is performed in reactive distillation columns, tubular reactors or fixed bed reactors, more preferably in a tubular reactor.

11. Process for preparing isophorone according to at least one of the preceding claims, **characterized in that** the removal of the individual fractions in stage 2 is performed by separation methods such as distillation, flash evaporation, crystallization, extraction, sorption, permeation, phase separation or combinations of the above, continuously or batchwise, in one or more stages, preferably by distillation in one or more apparatuses.

12. Process for preparing isophorone according to at least one of the preceding claims, **characterized in that** the removal in stage 2 is performed by distillation spatially separately from the isophorone synthesis or takes place in a reaction apparatus, preferably by means of a reactive distillation, preferably in a reactive distillation column.

13. Process for preparing isophorone according to at least one of the preceding claims, **characterized in that** the removal in stage 2 is performed spatially separately from the isophorone synthesis by a reactive distillation, preferably in a reactive distillation column, with a sidestream withdrawal.

14. Process for preparing isophorone according to at least one of the preceding claims, **characterized in that** fraction a) is withdrawn as a vapour stream comprising essentially acetone, water and low boilers, essentially diacetone alcohol and mesityl oxide, condensed and added again to the reactor with the acetone, water and catalyst feedstocks.

15. Process for preparing isophorone according to at least one of the preceding claims, **characterized in that** fraction b) is withdrawn as a sidestream of a distillation column, preferably of a reactive distillation column.

16. Process for preparing isophorone according to at least one of the preceding claims, **characterized in that** fraction b) is purified by a combination of neutralization or extraction and subsequent distillation in a reactive distillation column.

17. Process for preparing isophorone according to at least one of the preceding claims, **characterized in that** the worked-up phase of fraction b) is conducted into the hydrolysis with the products of value composed of isophorone and high boilers, with or without catalyst.

18. Process for preparing isophorone according to at least one of the preceding claims, **characterized in that** any further phase of fraction b) obtained, composed of products of value essentially comprising acetone, diacetone alcohol and mesityl oxide, is recycled into the reaction.

19. Process for preparing isophorone according to at least one of the preceding claims, **characterized in that** the hydrolysis c) is effected in a reactive distillation column.

20. Process for preparing isophorone according to at least one of the preceding claims, **characterized in that** the hydrolysis c) is effected at a water concentration of 0.1-99.9% by weight, preferably 30-90% by weight.

21. Process for preparing isophorone according to at least one of the preceding claims, **characterized in that** the hydrolysis c) is effected at catalyst concentrations of 0.001-10% by weight, more preferably of 0.05-1% by weight.

22. Process for preparing isophorone according to at least one of the preceding claims, **characterized in that** the hydrolysis c) is performed at a pressure in the hydrolysis reactor of 1-200 bar, preferably 20-60 bar, more preferably at least at the pressure which also exists in the isophorone synthesis step (reaction).

23. Process for preparing isophorone according to at least one of the preceding claims, **characterized in that** the hydrolysis c) is effected at a temperature of 100-300°C, preferably 210-260°C.

24. Process for preparing isophorone according to at least one of the preceding claims, **characterized in that** the organic fraction d) is purified by distillation, preferably by a combination of neutralization or extraction and subsequent distillation.

25. Process for preparing isophorone according to at least one of the preceding claims, **characterized in that** the hydrolysis 6 is effected at a water concentration of 0.1-99.9% by weight, preferably 30-90% by weight.

26. Process for preparing isophorone according to at least one of the preceding claims, **characterized in that** the hydrolysis 6 is effected at catalyst concentrations of 0.001-10% by weight, more preferably of 0.05-1% by weight.

27. Process for preparing isophorone according to at least one of the preceding claims, **characterized in that** the hydrolysis 6 is effected at a pressure in the hydrolysis reactor of 1-200 bar, preferably 20-60 bar.

28. Process for preparing isophorone according to at least one of the preceding claims, **characterized in that** the hydrolysis 6 is effected at a temperature of 100-300°C, preferably 210-260°C.

29. Process for preparing isophorone according to at least one of the preceding claims, **characterized in that** the hydrolysis 6 is performed in one or more stages in one or more apparatuses.

30. Process for preparing isophorone according to at least one of the preceding claims, **characterized in that** the onward passage of the vapours is effected from the top of the hydrolysis apparatus 6 into the hydrolysis apparatus 1.

31. Process for preparing isophorone according to at least one of the preceding claims, **characterized in that** the hydrolysis 6 is effected in at least one reactive distillation column.

32. Process for preparing isophorone according to at least one of the preceding claims, **characterized in that** the hydrolysis 6 is effected in one stage in a reactive distillation column.

33. Process for preparing isophorone according to at least one of the preceding claims, **characterized in that** a distillative workup of the aqueous fraction e) is effected and the vapours are passed from the top of the distillative workup apparatus onward into the hydrolysis apparatus 1, the distillative workup preferably being performed in one or more distillation columns.

34. Process for preparing isophorone according to Claim 33, **characterized in that** the wastewater obtained in the bottoms (stream f) is sent to a flash evaporation and separated further.

## Revendications

1. Procédé de fabrication d'isophorone par :
1. des condensations aldoliques catalysées d'acétone en tant que réactif ;
2. le traitement du produit de réaction,
le traitement lors de l'étape 2 ayant lieu en trois fractions :
a) une fraction constituée par de l'acétone non réagie, de l'eau et des composants de point d'ébullition faible, qui est condensée, puis recyclée pour la réaction dans le réacteur ;
b) une fraction, dans laquelle des substances colorantes sont notamment concentrées, cette fraction étant davantage purifiée et les substances de valeur contenues étant recyclées dans le procédé ;
c) une fraction constituée par de l'isophorone, des produits condensés supérieurs et de l'eau et un catalyseur, nommée courant de substances de valeur, cette fraction étant ensuite soumise à une hydrolyse 1, lors de laquelle des produits secondaires sont transformés en partie ou en totalité en isophorone, en acétone et en d'autres produits de valeur ;
3. la fraction traitée c) étant soumise à une séparation de phases en une fraction essentiellement organique d) et une fraction essentiellement aqueuse e) ;
4. le traitement par distillation de la fraction aqueuse e) et l'acheminement des vapeurs depuis la tête de l'appareil du traitement par distillation dans l'appareil d'hydrolyse 1 ;
5. la purification de la fraction organique d) en les trois fractions suivantes :
i) un résidu,
ii) de l'isophorone pure,
iii) des composants de point d'ébullition faible restants ;
6. l'hydrolyse 2 du résidu i) et le recyclage des produits de valeur formés dans le procédé.

2. Procédé de fabrication d'isophorone selon la revendication 1, **caractérisé en ce que** l'eau issue du fond du traitement par distillation de la fraction aqueuse e) est soumise à une évaporation avec détente, et l'eau purifiée formée est recyclée dans le procédé de fabrication d'isophorone.

3. Procédé de fabrication d'isophorone selon la revendication 1 ou 2, **caractérisé en ce que** la réalisation de la réaction a lieu dans la phase liquide, l'acétone étant transformée en isophorone par une réaction catalytique à des températures dans la plage allant de 100 à 250 °C, de préférence de 150 à 250 °C, de manière particulièrement préférée de 180 à 250 °C, et dans une plage de pression allant de 5 à 50 bar, de préférence de 10 à 50 bar, de manière particulièrement préférée de 20 à 50 bar, les valeurs indiquées pouvant être combinées de manière quelconque les unes avec les autres.

4. Procédé de fabrication d'isophorone selon la revendication 1 ou 2, **caractérisé en ce que** la réalisation de la réaction a lieu dans la phase gazeuse, l'acétone étant transformée en isophorone par une réaction catalytique à des températures dans la plage allant de 100 à 400 °C, de préférence de 200 à 400 °C.

5. Procédé de fabrication d'isophorone selon la revendication 1 ou 2, **caractérisé en ce que** la réalisation a lieu dans la plage supercritique, l'acétone étant transformée en isophorone par une réaction catalytique à des températures dans la plage allant de 250 à 350 °C et dans une plage de pression allant de 50 à 200 bar.

6. Procédé de fabrication d'isophorone selon au moins l'une quelconque des revendications précédentes, **caractérisé en ce qu'**un catalyseur homogène ou hétérogène, de préférence un catalyseur homogène, est utilisé.

7. Procédé de fabrication d'isophorone selon au moins l'une quelconque des revendications 1 à 3 précédentes, **caractérisé en ce que** la réaction a lieu dans la phase liquide avec un catalyseur homogène.

8. Procédé de fabrication d'isophorone selon la revendication 7, **caractérisé en ce que** des quantités d'alcalis < 1 % en poids, de préférence < 0,2 % en poids, sont utilisées en tant que catalyseur, de préférence NaOH en des quantités de 0,015 à 0,05 % en poids.

9. Procédé de fabrication d'isophorone selon au moins l'une quelconque des revendications précédentes, **caractérisé en ce que** la réaction est réalisée dans des réacteurs choisis parmi les réacteurs tubulaires, les cuves agitées, les cascades de cuves agitées, les réacteurs à lit fixe, les colonnes de distillation réactives, les réacteurs microstructurés, les réacteurs à boucle ou dans des combinaisons de ces réacteurs.

10. Procédé de fabrication d'isophorone selon au moins l'une quelconque des revendications précédentes, **caractérisé en ce que** la réaction est réalisée dans des colonnes de distillation réactives, des réacteurs tubulaires ou des réacteurs à lit fixe, de manière particulièrement préférée dans un réacteur tubulaire.

11. Procédé de fabrication d'isophorone selon au moins l'une quelconque des revendications précédentes, **caractérisé en ce que** la séparation des fractions individuelles à l'étape 2 est réalisée par des méthodes de séparation telles que la distillation, l'évaporation avec détente, la cristallisation, l'extraction, la sorption, la perméation, la séparation de phases ou des combinaisons des méthodes mentionnées, de manière continue ou discontinue, en une ou plusieurs étapes, de préférence par distillation dans un ou plusieurs appareils.

12. Procédé de fabrication d'isophorone selon au moins l'une quelconque des revendications précédentes, **caractérisé en ce que** la séparation à l'étape 2 est réalisée par distillation séparément dans l'espace de la synthèse d'isophorone ou a lieu dans un appareil de réaction, de préférence par une distillation réactive, de préférence dans une colonne de distillation réactive.

13. Procédé de fabrication d'isophorone selon au moins l'une quelconque des revendications précédentes, **caractérisé en ce que** la séparation à l'étape 2 est réalisée séparément dans l'espace de la synthèse d'isophorone par une distillation réactive, de préférence dans une colonne de distillation réactive, avec un soutirage de courant latéral.

14. Procédé de fabrication d'isophorone selon au moins l'une quelconque des revendications précédentes, **caractérisé en ce que** la fraction a) est soutirée en tant que courant de vapeur, contenant essentiellement de l'acétone, de l'eau et des composants de point d'ébullition faible, essentiellement de l'alcool diacétonique et de l'oxyde mésitylique, condensée et réintroduite dans le réacteur avec les matières premières acétone, eau et le catalyseur.

15. Procédé de fabrication d'isophorone selon au moins l'une quelconque des revendications précédentes, **caractérisé en ce que** la fraction b) est soutirée en tant que courant latéral d'une colonne de distillation, de préférence d'une colonne de distillation réactive.

16. Procédé de fabrication d'isophorone selon au moins l'une quelconque des revendications précédentes, **caractérisé en ce que** la purification de la fraction b) a lieu par une combinaison d'une neutralisation ou d'une extraction, et d'une distillation ultérieure dans une colonne de distillation réactive.

17. Procédé de fabrication d'isophorone selon au moins l'une quelconque des revendications précédentes, **caractérisé en ce que** la phase traitée de la fraction b) est introduite dans l'hydrolyse avec les produits de valeur constitués par de l'isophorone, des composants de point d'ébullition élevé et éventuellement le catalyseur.

18. Procédé de fabrication d'isophorone selon au moins l'une quelconque des revendications précédentes, **caractérisé en ce qu'**une autre phase obtenue de la fraction b) constituée par des produits de valeur, contenant essentiellement de l'acétone, de l'alcool diacétonique et de l'oxyde mésitylique, est recyclée dans la réaction.

19. Procédé de fabrication d'isophorone selon au moins l'une quelconque des revendications précédentes, **caractérisé en ce que** l'hydrolyse c) a lieu dans une colonne de distillation réactive.

20. Procédé de fabrication d'isophorone selon au moins l'une quelconque des revendications précédentes, **caractérisé en ce que** l'hydrolyse c) a lieu à une concentration en eau dans l'hydrolyse de 0,1 à 99,9 % en poids, de préférence de 30 à 90 % en poids.

21. Procédé de fabrication d'isophorone selon au moins l'une quelconque des revendications précédentes, **caractérisé en ce que** l'hydrolyse c) a lieu à des concentrations en catalyseur de 0,001 à 10 % en poids, de manière particulièrement préférée de 0,05 à 1 % en poids.

22. Procédé de fabrication d'isophorone selon au moins l'une quelconque des revendications précédentes, **caractérisé en ce que** l'hydrolyse c) est réalisée à une pression dans le réacteur d'hydrolyse de 1 à 200 bar, de préférence de 20 à 60 bar, de manière particulièrement préférée au moins à la pression qui règne également dans l'étape de synthèse d'isophorone (réaction).

23. Procédé de fabrication d'isophorone selon au moins l'une quelconque des revendications précédentes, **caractérisé en ce que** l'hydrolyse c) est réalisée à une température de 100 à 300 °C, de préférence de 210 à 260 °C.

24. Procédé de fabrication d'isophorone selon au moins l'une quelconque des revendications précédentes, **caractérisé en ce que** la purification de la fraction organique d) a lieu par distillation, de préférence par une combinaison d'une neutralisation ou d'une extraction et d'une distillation ultérieure.

25. Procédé de fabrication d'isophorone selon au moins l'une quelconque des revendications précédentes, **caractérisé en ce que** l'hydrolyse 6. a lieu à une concentration en eau dans l'hydrolyse de 0,1 à 99,9 % en poids, de préférence de 30 à 90 % en poids.

26. Procédé de fabrication d'isophorone selon au moins l'une quelconque des revendications précédentes, **caractérisé en ce que** l'hydrolyse 6. a lieu à des concentrations en catalyseur de 0,001 à 10 % en poids, de manière particulièrement préférée de 0,05 à 1 % en poids.

27. Procédé de fabrication d'isophorone selon au moins l'une quelconque des revendications précédentes, **caractérisé en ce que** l'hydrolyse 6. est réalisée à une pression dans le réacteur d'hydrolyse de 1 à 200 bar, de préférence de 20 à 60 bar.

28. Procédé de fabrication d'isophorone selon au moins l'une quelconque des revendications précédentes, **caractérisé en ce que** l'hydrolyse 6. est réalisée à une température de 100 à 300 °C, de préférence de 210 à 260 °C.

29. Procédé de fabrication d'isophorone selon au moins l'une quelconque des revendications précédentes, **caractérisé en ce que** l'hydrolyse 6. est réalisée dans un ou plusieurs appareils en une étape ou en plusieurs étapes.

30. Procédé de fabrication d'isophorone selon au moins l'une quelconque des revendications précédentes, **caractérisé en ce que** l'acheminement des vapeurs a lieu depuis la tête de l'appareil d'hydrolyse 6. dans l'appareil d'hydrolyse 1.

31. Procédé de fabrication d'isophorone selon au moins l'une quelconque des revendications précédentes, **caractérisé en ce que** l'hydrolyse 6. a lieu dans au moins une colonne de distillation réactive.

32. Procédé de fabrication d'isophorone selon au moins l'une quelconque des revendications précédentes, **caractérisé en ce que** l'hydrolyse 6. a lieu en une étape dans une colonne de distillation réactive.

33. Procédé de fabrication d'isophorone selon au moins l'une quelconque des revendications précédentes, **caractérisé en ce qu'**un traitement par distillation de la fraction aqueuse e) a lieu et un acheminement des vapeurs depuis la tête de l'appareil de traitement par distillation dans l'appareil d'hydrolyse 1, le traitement par distillation étant de préférence réalisé dans une ou plusieurs colonnes de distillation.

34. Procédé de fabrication d'isophorone selon la revendication 33, **caractérisé en ce que** l'eau résiduaire formée dans le fond (courant f) est introduite dans une évaporation avec détente et davantage séparée.
